(19) **Europäisches Patentamt**
**European Patent Office**
**Office européen des brevets**

(11) **EP 1 206 290 B1**

(12) **EUROPEAN PATENT SPECIFICATION**

(45) Date of publication and mention
of the grant of the patent:
**28.11.2007 Bulletin 2007/48**

(51) Int Cl.:
*A61L 15/58* (2006.01)    *A61L 24/00* (2006.01)
*A61L 24/08* (2006.01)    *A61L 26/00* (2006.01)

(21) Application number: **00948170.6**

(22) Date of filing: **27.07.2000**

(86) International application number:
**PCT/GB2000/002895**

(87) International publication number:
**WO 2001/013968 (01.03.2001 Gazette 2001/09)**

(54) **CYCLODEXTRIN CONTAINING PRESSURE SENSITIVE ADHESIVES**

CYCLODEXTRIN ENTHALTENDE HAFTKLEBSTOFFE

ADHESIFS AUTOCOLLANTS CONTENANT DE LA CYCLODEXTRINE

(84) Designated Contracting States:
**AT BE CH CY DE DK ES FI FR GB GR IE IT LI LU MC NL PT SE**

(30) Priority: **25.08.1999 GB 9920167**

(43) Date of publication of application:
**22.05.2002 Bulletin 2002/21**

(73) Proprietor: **AVERY DENNISON CORPORATION Pasadena, CA 91103 (US)**

(72) Inventor: **LIPMAN, Roger, David, Arnold B-2360 Oud-Turnhout (BE)**

(74) Representative: **Bankes, Stephen Charles Digby et al BARON & WARREN 19 South End Kensington London W8 5BU (GB)**

(56) References cited:
WO-A-97/39742          WO-A-99/64485
US-A- 4 192 785         US-A- 4 231 369

• DATABASE WPI Section Ch, Week 198419 Derwent Publications Ltd., London, GB; Class A96, AN 1984-117671 XP002149919 & JP 59 055825 A (NITTO ELECTRIC IND CO), 31 March 1984 (1984-03-31)
• PATENT ABSTRACTS OF JAPAN vol. 012, no. 344 (C-528), 16 September 1988 (1988-09-16) & JP 63 101316 A (NICHIBAN CO LTD), 6 May 1988 (1988-05-06)
• DATABASE WPI Section Ch, Week 199136 Derwent Publications Ltd., London, GB; Class A81, AN 1991-262350 XP002149920 & JP 03 170575 A (SEKISUI CHEM IND CO LTD) , 24 July 1991 (1991-07-24)
• DATABASE WPI Section Ch, Week 198333 Derwent Publications Ltd., London, GB; Class A96, AN 1983-737343 XP002149921 & JP 58 113275 A (NICHIBAN KK), 6 July 1983 (1983-07-06)
• DATABASE WPI Section Ch, Week 199427 Derwent Publications Ltd., London, GB; Class A92, AN 1994-222253 XP002149922 & JP 06 158002 A (SEKISUI PLASTICS CO LTD) , 7 June 1994 (1994-06-07) cited in the application
• DATABASE WPI Section Ch, Week 198145 Derwent Publications Ltd., London, GB; Class A96, AN 1981-82783D XP002149923 & JP 56 123912 A (NITTO ELECTRIC IND CO), 29 September 1981 (1981-09-29)

**Description**

This invention relates to pressure sensitive adhesive compositions containing cyclodextrins. By the term "pressure sensitive adhesive" is meant those adhesives that have touch perceivable tack, that are easily deformed in the time scale allowed for bond formation and that are capable of being applied and bonded to the substrate at temperatures no higher than about 50°C, and preferably at ambient temperatures. Pressure sensitive adhesives are employed in many fields. Pressure sensitive adhesives are used, inter alia, as components of labels, industrial tapes, postage stamps, stationery products, and in medical products such as surgical drapes, tapes, ostomy products, wound care products and devices for transdermal drug delivery.

This invention relates particularly to pressure sensitive adhesives used in contact with skin and more particularly to the class of medical pressure sensitive adhesives that are generally termed hydrocolloid adhesives. Most particularly, the invention relates to adhesives that are able to absorb odours associated with body fluids and/or wounds.

Hydrocolloid adhesives are a unique kind of medically useful pressure sensitive adhesive. They have usually two phases - a rubbery phase which provides pressure sensitive tack, sometimes called "dry tack" and, dispersed within the continuous rubbery phase, a discontinuous phase of absorbent material. Depending upon the nature of the absorbents, and especially whether the absorbent is soluble in aqueous media or merely swellable, the adhesive composition can develop "wet tack" as it becomes imbibed with fluid. Such wet tack can also influence the adhesive power of the hydrocolloid. Hydrocolloid adhesives thus have a duality of attributes in that they are inherently adhesive and inherently absorbent. They are useful as wound dressings because they can be applied directly to open wounds and can be secured on the surrounding intact skin, and as skin barriers because they protect the peristomal skin of ostomy patients. Hydrocolloid adhesives maintain the skin in a normally or optimally hydrated condition. Optimally hydrated skin is less subject to irritation and injury from repeated application and removal of adhesives than is macerated skin, which latter can result from the use of conventional pressure sensitive adhesives on the skin.

Many hydrocolloid skin barriers are known and are used especially in the fields of ostomy care and wound care. It is convenient to divide these into "integrated" compositions and "non-integrated" compositions. In this context, "integrated" means those compositions that substantially retain their dimensional stability and form when saturated with wound exudate and/or other body fluid. Integrated hydrocolloids usually contain cross-linked or pseudo cross-linked rubbery matrices in the continuous phase to provide the integrating network. But specific combinations of absorbents have also been reported to give highly integrated hydrocolloids, even in the absence of a cross-linked rubbery matrix. "Non-integrated" means those compositions which become soft gels and amorphous as they become saturated with fluid. In this invention, both integrated and non-integrated hydrocolloids are encompassed.

It is often desired to add small quantities of an agent to a medical pressure sensitive adhesive to confer additional benefits. For example, skin problems are common for persons who have a stoma, which is an artificial body opening produced as a result of surgery. PCT Application WO 98/01167 discloses in this regard a hydrocolloid pressure sensitive adhesive containing an amount of aloe vera up to 5wt% which is said to offer a better protection of the skin against the aggressive action of excreted substances. Again, in European Patent Specification No. 0 023 395 B1, mention is made of pressure sensitive adhesives containing a broad spectrum antimicrobial agent. In this prior art, the antimicrobial agent is dissolved in a suitable solvent and dispersed in the said pressure sensitive adhesive to give a two-phase system, which is said to release the antimicrobial agent in a controlled way.

If the context permits, we use in this Application the term "hydrocolloid" to embrace the term "cyclodextrin".

According to a first aspect of the present invention there is provided a medical or surgical appliance in the form of a wound dressing or an ostomy appliance or a skin barrier, comprising a substrate on which is formed a layer of pressure sensitive adhesive composition, the pressure sensitive adhesive composition comprising a rubbery continuous phase with a discontinuous phase distributed therein, characterised in that the discontinuous phase comprises 0.1 to 65 wt %, based on the total composition, of a hydrocolloid composition comprising an uncomplexed cyclodextrin, and a hydrocolloid other than cyclodextrin.

The cyclodextrin-containing hydrocolloid adhesive may contain an effective amount of one or more active ingredients. Hydrocolloid adhesives are particularly suitable as the basis for such constructions, because of their ability to maintain the skin in a normally or optimally hydrated condition. Often, such skin patches will be applied to the skin over an extended period, and hydrocolloid adhesives will maintain the skin in a healthy condition. As non-limiting examples of what is contemplated, the active ingredient may be an antibacterial or antifungal compound, a compound such as hydroquinone, a compound such as an essential oil, for example tea tree oil, or mixtures of essential oils, a compound such as salicylic acid, a compound such as menthol, or a fragrance composition.

Non-limiting examples of prior art hydrocolloid compositions suitable for use as bases for the reduction to practice of the present invention, with appropriate modification in accord with the teachings herein described, are given in US Patent 3,339,546, US Patent 4,231,369, US Patent 4,477,325, US Patent 4,738,257, US Patent 4, 551, 490, US Patent 4,192,785 and US Patent 4, 952, 618.

The present invention also provides skin barriers and wound dressings comprising a layer of hydrocolloid adhesive containing an effective amount of a cyclodextrin and which is backed by a non-adhesive, waterproof film to act as a skin barrier. The skin barrier is used in a number of ways. One of these is for wound dressing purposes. Patients in institutional settings such as hospitals and nursing homes often have or acquire chronic wounds such as venostasis ulcers and bed sores, and these wounds can possess a very offensive odour. Bandages or dressings made from or incorporating the compositions of the invention are able to absorb odour molecules and thereby reduce or eliminate the offensive odour. This contributes to the well-being of the patient as well as to the nursing staff and other patients, since the odours from chronic wounds can be offensive to both carers and to family members of the patient. Another important use is for the protection of the skin around body openings, especially around the surgically created openings known as colostomies, ileostomies and urostomies. Collectively, these surgically created body openings are often termed stomas. The novel appliances of the invention are able to absorb the odour molecules that are associated with faeces and urine and thus are able to assist in the control of the odour that is associated with stomas. This can increase the self-confidence of the patient because such odours can be a source of personal embarrassment. While not wishing to be bound by any particular theory, it is believed that the cyclodextrin molecules are able to absorb malodorous molecules, which become complexed within the toroidal structure of the cyclodextrin molecule.

One yet further aspect of the invention relates to incorporation of cyclodextrin materials into pressure sensitive adhesives based on hydrophilic polymers, which will usually, though not necessarily, be crosslinked. These materials are commonly termed hydrogels, and can form the basis of medical pressure sensitive adhesives. With hydrogels, the pressure sensitive adhesive property is achieved by plasticisation of the polymer with water and/or a hydrophilic plasticiser, and thus the cyclodextrin may or may not be completely dissolved in the pressure sensitive adhesive, depending on whether the cyclodextrin is completely soluble in the plasticising medium. Thus, the possibility exists with this adhesive system for a cyclodextrin containing pressure sensitive adhesive that has only one phase.

The introduction of cyclodextrins, in combination with certain "active compounds", into pressure sensitive adhesives is known and is reported in the prior art.

For example Japanese Patent Application JP 6158002A, to Sekisui Plastics Co., describes an antibacterial pressure sensitive adhesive for food containers, wherein isothiocyanuric acid ester is complexed within β-cyclodextrin and added to a pressure sensitive adhesive which is then adhered to the inside surface of the food container to control bacterial and fungal growth.

US Patent 5,352,717 to American Maize Technology Inc. teaches adhesive or sealant compositions containing a small amount of cyclodextrin. Some examples also have, as an optional component, a blowing agent to expand the adhesive to a foamed material for industrial uses. Although one of the examples talks to a "pressure sensitive adhesive", the exemplified adhesive is said to be applied at temperatures of 160°C., as are the other examples, which are clearly directed at hot melt adhesive compositions, outside the contemplated area of interest of the instant invention.

US Patent 4,978,532 to Pharmedic Co. describes a dosage form for the administration of dehydroepiandrosterone (DHEA). The dosage form comprises a medical grade silicone pressure sensitive adhesive, DHEA and a permeation enhancer, an example of which is hydroxypropyl-β-cyclodextrin.

Also of interest is the US Patent 5,562,917 to Pentech Pharm Inc. that claims a composition suitable for the treatment of Parkinson's disease involving the transdermal delivery of apomorphine in an aqueous based gel. Specified is a permeation enhancer which includes hydroxypropyl-β-cyclodextrin. Also specified is a matrix of silicone copolymer pressure sensitive adhesive, 0.1 - 3wt% of apomorphine and a carbocyclic compound having pendant hydroxyl groups selected from butylated hydroxy anisole, butylated hydroxy toluene and hydroxypropyl-β-cyclodextrin as a permeation enhancer.

The use of cyclodextrins in cosmetics as odour absorbing agents and as vehicles to deliver active ingredients to, for example, the skin, is known in the literature. For example, PCT Applications WO 98/56343 WO 98/56344 and WO 98/56345 disclose cosmetic compositions for topical application to the skin that contain an aqueous phase or a water-in-silicone emulsion, a cyclodextrin compound and a salicylic acid derivative. Such compositions are said to provide anti-acne and anti-inflammatory activity together with reduced skin irritation.

PCT Application WO 99/06078 discloses cyclodextrin odour absorbent articles for decreasing odours associated with body fluids, where the cyclodextrin has a specific particle size. PCT Application WO 98/56890 teaches the combination of aqueous cyclodextrin compositions with wrinkle control agents for odour and wrinkle control of fabrics. PCT Applications WO 98/17239, WO 98/17240, WO 98/56341 and WO 98/56342 describe an aqueous carrier containing soluble cyclodextrins, perfume compositions and preferably hydrophobic antimicrobial compounds in order to reduce body odour and or environmental odour. PCT Applications WO 98/18439 and WO98/5634 reveal similar compositions in a powder carrier such as a starch. PCT Application WO 98/56339 describes similar compositions in a two phase formulation comprised of an aqueous phase and an oil phase.

US Patent 5,429,628 and PCT Application WO 94/22501 both teach the preparation of articles containing small particle size cyclodextrin for odour control. A large number of consumer absorbent products, such as panty liners, catamenials, diapers and sanitary napkins are exemplified, where the cyclodextrin is present within, or as part of, a fibrous absorbent medium.

European Patent Specification 0 392 608 B1 concerns solid consumer product compositions containing an ingredient such as a flavour, a drug or a perfume that is complexed within a cyclodextrin. Product categories that are mentioned include those such as drinks and beverages, canned pet foods, cosmetics and toiletries such as facial scrubs, body powders, depilatories, mouth washes and lipsticks, detergents, paper towels, cake mixes and cookies, incense, room deodorant blocks, dyes, insect repellents and the like.

PCT Patent Application WO 94/18973 discloses the complexing of bisacodyl with cyclodextrin to provide an oral dosage form that delays the laxative action of the bisacodyl until the preparation is near the junction of the small intestine and the colon.

US Patent 5,246,611 and PCT Applications WO 93/05136 and WO 91/17300 all deal with the use of cyclodextrin perfume complexes in fabric treatment products such as fabric softeners. The cyclodextrin complexes are suspended in polyalkylene glycol carrier which does not prematurely displace the active from the cyclodextrin.

What becomes evident from a reading of the prior art is that, notwithstanding the very great amount of art that exists concerning cyclodextrin containing materials, nowhere in the literature is reported the use of cyclodextrins as an integral component in pressure sensitive adhesive compositions.

Concerning odour absorbent adhesives, there appears to be little or no prior art. Japanese Application 10168348A to Kodaijin Sugaoka KK does disclose incorporation of cristobalite as a component of wall coatings which also contain pressure sensitive adhesives, and the cristobalite is said to absorb foul odours. But the finished composition is not itself pressure sensitive adhesive; indeed, it is said to resemble Japanese lime plaster.

McNeil-PPC Inc. in US Application 93168550 A disclose panty liners in which a pressure sensitive adhesive is applied to an absorbent structure containing baking soda to absorb odours. Plasto SA, in German Application 19724871 A1, claim a laminated structure comprised of a polyolefin film having dispersed in it a perfume, and coated on one side with a pressure sensitive adhesive. Such a structure will obviously serve only to mask an odour, and the perfume is in the film, not in the adhesive. Schering Plough in US Patent 5,399,404 teach a patch for masking foot odours comprising a carrier on one side of a non-occlusive layer which contains fragrance and a pressure sensitive adhesive on the other side of the carrier to secure the patch to the foot or shoe. In PCT WO 9423766 A1, Schering Plough also disclose a deodoriser for masking foot and shoe odours comprised of a pressure sensitive adhesive laminated to a felt which contains a perfume. Kock in Canadian Application 2041278 A describes a disposable perspiration absorbing pad which can be adhered to clothing and adjusted in size and shape and which contains an odour absorbing or perfumed layer.

In none of the above prior art is there any reference or suggestion that the pressure sensitive adhesive component is itself odour absorbing.

What is surprising and unexpected in the instant invention is that, if cyclodextrins are employed as the integral absorbant filler, or as part of the integral absorbent fillers, in hydrocolloid adhesives, the resulting compositions have a unique and wide versatility. They are capable of adhering to surfaces, absorbing fluids, absorbing odours, providing a fragrance and delivering active substances. There is no indication or suggestion in the literature to add or incorporate cyclodextrins into hydrocolloid adhesives, nor any indication or suggestion that such addition should yield the advantageous results of odour control and reduction.

The adhesive of the invention comprises any suitable pressure sensitive adhesive matrix known in the art and having hydrocolloid particles and cyclodextrins dispersed or dissolved therein. The permanently tacky pressure sensitive adhesive component must be tacky at room temperature as well as at the skin temperature of patients. Also, the adhesive must be dermatologically acceptable, which means that after continuous contact with skin there is little adhesive residue upon removal and there is no significant reaction with the skin during the adhesion period. The adhesive strength of the continuous phase must be sufficient to adhere to the skin of the patient for the time determined by the use of the medical device of which the adhesive forms part. Other ingredients such as tackifiers, plasticisers, and polymer stabilisers may be added to the continuous rubbery phase, to modify tack and optimise adhesion properties and to protect polymers from degradation during processing.

The adhesive matrix may be based on for example polyisobutylene, butyl rubber, polyacrylates, polyurethanes, silicone gum, natural gum rubber, SBR rubber or polyvinyl ether. Thermoplastic elastomers such as styrene-isoprene-styrene block copolymers and styrene-ethylene/propylene-styrene block copolymers may be used, and these may require optional tackifiers and plasticisers. Blends or mixtures of elastomers may be employed. Conventional additives such as tackifiers, softeners, plasticisers and antioxidants may be present to modify, adjust and stabilise the adhesive and other properties of the matrix. The amount of adhesive matrix with respect to the total composition will generally be from 35wt% to about 99wt% or more. Hydrophilic polymers, such as are used in hydrogels, may also form the basis of the pressure sensitive adhesive matrix.

Suitable hydrocolloids for use in the adhesives in conjunction with the cyclodextrins are naturally occurring hydrocolloids such as pectins, guar gum, karaya gum, locust bean gum, carageenan, tragacanth gum, alginates, xanthan gum, modified naturally derived substances such as sodium carboxymethyl cellulose, synthetic materials such as polvinylalcohol, polyoxyalkylene polyols, polyvinyl pyrollidone, and animal derived materials such as gelatine. Ionic hydrocolloids such as hyaluronic acid, chitosan salts or DEAE Dextran may also be employed. The hydrocolloids may be water absorbable or water swellable, and combinations of one type or of various types may be used in any ratio. A hydrocolloid is included in addition to cyclodextrin, and may be used in an amount up to 60wt% or more. Combined with the cyclodextrin component the aggregate of the two will amount to from 0.1wt% to 65wt%.

The term cyclodextrin, as used herein, includes any of the known cyclodextrins. Cyclodextrin materials are cyclic oligosaccharides containing a minimum of six D-(+)-glucopyranose units attached by $\alpha$ - (1 > 4) glucosidic bonds. Three cyclodextrins called $\alpha$, $\beta$ and y are naturally occurring and have, respectively, six, seven and eight glucose units. Cyclodextrins are known that contain up to twelve glucose units. Cyclodextrin materials can also be manufactured from starch by enzymatic degradation. In addition, many synthetic modifications of the natural material materials are known, for example methyl-$\beta$-cyclodextrin and hydroxypropyl-$\beta$-cyclodextrin. The conformations of the cyclic structures of these molecules are such that the molecules are arranged in rigid conical molecular shapes that have hollow interiors of very well defined sizes. These internal cavities are hydrophobic in nature because the interior of the toroidal shape is predominantly made up of hydrogen atoms. The interior shapes of the cyclodextrins are able to form inclusion complexes, sometimes referred to as "host-guest" complexes, or clathrate compounds, with organic molecules which fit, completely or partially, into the cavities defined by the toroidal shapes. For example, odiferous molecules can fit into the cavities. This includes both perfumes and malodorous compounds. Cyclodextrins therefore, and especially mixtures of cyclodextrins with cavities of different sizes, can be used to control odours. Uncomplexed or free cyclodextrins, dispersed within the adhesive matrix, are used to absorb malodours. The complexation of odorous molecules by cyclodextrin is facilitated by the presence of water. It will be understood that the water necessary to facilitate such complexing of malodour by the cyclodextrin is present in the contaminating urine or faeces, and/or is released by the skin through normal transpiration, and will be absorbed by the adhesive.

The choice of cyclodextrin employed in a given formulation will be decided on the basis of the properties desired in the finished product, and the specific role that the cyclodextrin is fulfilling. Unmodified $\beta$-cyclodextrin is not very water soluble and is generally not preferred if high absorbancy is needed. $\alpha$-cyclodextrins, $\gamma$-cyclodextrins and certain modified $\beta$-cyclodextrins are more water absorbent. Mixtures of cyclodextrins are often preferred, because these will absorb a wider range of malodorous molecules than will a single cyclodextrin. The cyclodextrin to be used for a specific complex will of course be determined by the size and shape of the active molecule to be complexed.

Any active ingredient may be considered for addition to the formulations anticipated by the instant invention. By active ingredient, we mean an ingredient that is not essential to the functioning of the formulation as a moisture and odour absorbing pressure sensitive adhesive. An active ingredient is added to confer an additional benefit to the formulation. The following active ingredients exemplify the scope of the invention, and represent a non-limiting list of active ingredients.

Aspirin, benzocaine, benzyl alcohol, butamben picrate, camphor, camphorated metacresol, chloral hydrate, chlorabutanol, chloraxylenol, cyclomethycaine sulphate, dibucaine, dibucaine hydrochloride, dimethisoquin hydrochloride, diphenhydramine hydrochloride, dyclonine hydrochloride, eugenol, glycol salicylate, hexyl resorcinol, hydrocortisone, hydrocortisone acetate, juniper tar, lidocaine, lidocaine hydrochloride, menthol, methapyrilene hydrochloride, phenol, phenolate sodium, pramoxine hydrochloride, resorcinol, resorcinol monoacetate, salicylamide, tetracaine, tetracaine hydrochloride, thymol, triethanolamine salicylate, tripelennamine hydrochloride, allyl isothiocyanate, ammonia, capsaicin, eucalyptus oil, histamine dihydrochloride, methyl nicotinate, methyl salicylate, turpentine oil, allantoin, calamine, dimethicone, glycerin, kaoline, petrolatum, shark liver oil, zinc acetate, zinc carbonate, zinc oxide, hydroquinone, quinine sulphate, vitamine E, pregnenolone acetate, progesterone, salicylic acid, clioquinol, haloprogin, miconazole nitrate, tolnaftate, undecylenic acid, benzoyl peroxide, sulphur, povidone iodine, benzalkonium chloride, benzethonium chloride, methylbenzethonium chloride, trichlosan, trichlocarban, chlorhexidine gluconate, bacitracin zinc, neomycin sulphate, glycolic acid, tea tree oil, lavender oil.

Active ingredients must be present at sufficient concentrations to achieve the desired effect. In general, however, active ingredients will be present usually at no greater than lowt%, and preferably at no greater than 5wt%, with respect to the total composition.

Other components such as chemical agents that facilitate release of active ingredients from the adhesive formulations, for example plasticisers and solvents for the active ingredients be optionally be present. Also agents that promote absorption of active ingredients by the skin, may optionally be added to the formulation. Non-limiting examples of such skin permeation enhancers are isopropyl myristate, oleic acid, propylene glycol and laurocapram. Other optional ingredients such as small amounts of pigments or colourants may also be present in the compositions.

Test Methods

The formulations in the examples below were evaluated using the following test methods.

Reverse tack

Reverse tack of hydrocolloid adhesives is the maximum force necessary to remove a standard polyester strip brought into contact with the hydrocolloid without external force, from this hydrocolloid surface.

Procedure

Make the test panel self adhesive using double coated tape. Laminate the hydrocolloid adhesive on the test panel. Place the test panel with hydrocolloid in the lower clamp of a tensile testing machine. Program the tensile tester. Place a polyester test strip of thickness 125 $\mu$ (5 mils) and dimensions (21 cm x 2.54 cm) in the upper clamp, making sure that the total length of polyester under the clamp (loop) is 15 cm. Remove the release liner from hydrocolloid and start the measurement.

The reverse tack is the maximum force to remove the polyester strip from the hydrocolloid surface.

90° Peel adhesion of hydrocolloid adhesives on SS

Peel adhesion on stainless steel (SS) is the average force to remove a hydrocolloid adhesive, laminated under specified conditions on a SS panel, from the SS panel at constant speed and at an angle of 90°.

Procedure

Clean the SS-panel with solvent. Cut a hydrocolloid sample of 25.4mm width and reinforce with reinforcing tape, laminate a paper strip at one end of the hydrocolloid sample using an overlap of about 1 cm. Remove the liner from the hydrocolloid sample and laminate the sample on the SS-panel with a 450 gm. roller at a speed of 150 cm/min. Allow the sample to dwell for 1 minute. Place the paper strip in the upper clamp and the SS-panel on the lower clamp, making sure that the angle between peel direction and SS-panel is 90°. Start the measurement using a crosshead speed of 300mm/min. The angle must be kept 90° until the measurement is completed. The 90° peel adhesion is the average force to remove the hydrocolloid strip from the SS-panel.

Static shear of hydrocolloid adhesives

Static shear is the time necessary to remove a hydrocolloid adhesive, laminated on a stainless steel panel under specified conditions, from the test panel under influence of a specified weight.

Procedure

Condition the hydrocolloid samples at 23 $\pm$ 1° and 50 $\pm$ 2% relative humidity for 24 hours. Clean the SS shear panel with solvent. Cut a hydrocolloid strip of 25.4 mm width and 50 mm length. Reinforce the hydrocolloid strip with reinforcing tape. Laminate the hydrocolloid strip on the test panel using an overlap surface of 1 inch$^2$. Protect the free hydrocolloid with release liner. Put a weight of 500 g on the laminate for 1 hour. Reinforce the free hydrocolloid adhesive zone with reinforcing plastic and perforate. Place the test panel with hydrocolloid on the shear bar using a shear weight of 500 g. Re-zero the registration-clock. Note the time on the clock when sample falls off under the influence of the 500g. Weight. This completes the measurement.

Static absorption of hydrocolloids.

To determine the amount of fluid uptake into a known surface of hydrocolloid adhesive.

Procedure

Laminate release liner to the upper flange of a moisture vapour transmission determination cup with the double coated tape. This is the contact zone for the hydrocolloid. Fill the cup with 30 ml NaCl solution (0.9%wt). Cut a sample of hydrocolloid of about the same size as the outer cup diameter. Weigh the sample ($W_1$). Laminate the sample to the cup, making sure that the seal between the hydrocolloid sample and the cup is water tight. Turn the cup upside down and put it in the oven at 37°C. for 24 hours. Cool down. Remove the hydrocolloid from the cup and reweigh ($W_2$). Calculate the water fluid absorption (g/sq.m.24h) using the formula :

$$abs = (W_2 - W_1) / 0.002375$$

where the area of the hydrocolloid in contact with salt solution is 0.002375 sq.m.

Determination of cold flow

The flow of the hydrocolloid under influence of a specified pressure and after a specified time, is measured.

Procedure

Condition the hydrocolloid samples at $23 \pm 1°C$ and $50 \pm 2$ % relative humidity for 24 hours. Cut 5 samples of hydrocolloid using a 35mm circular die-cutter. Put a silicone paper on top of a first glass plate. Arrange the 5 samples on the silicone paper in a way that pressure is distributed equally. Measure the diameter of each sample with callipers, mark the exact place where the measurement is done. Put a plastic disk on each sample. Put another silicone paper and two glass plates over the construction followed by a weight of 10 kg. (The measurement can also be done by placing the sample with the disk and the 10kg. weight in an oven maintained at 40°C). After 24 hours, measure the diameter of the samples where they are marked. Calculate the % increase of diameter of the samples. The cold flow is the % increase of diameter after 24 hours exposure to 10 kg (for 5 samples). Record the % increase in diameter and the test temperature.

Determination of the integrity of hydrocolloids

The integrity of a hydrocolloid is defined as its ability to resist breakdown by biological fluids. The test measures the weight percentage of hydrocolloid adhesive retained after exposure to saline under specified conditions.

Procedure

Condition the hydrocolloid samples at $23 \pm 1°C$ and $50 \pm 2$ % relative humidity for 2.4 hours. Cut circular samples 2.54cm in diameter from hydrocolloid sheet. Weigh and record the samples ($W_i$). Place each sample in a bottle with 50ml aqueous saline (0.9%wt). Cap the bottles and agitate on the bottle shaker at 400 speed for a period of 18hrs. Remove the sample and dry it in the circulating air oven at 50°C and 50% relative humidity until dry. This takes about 24 hours. Weigh and record the sample ($W_f$). The Integrity Value of the sample is calculated using the following equation:

$$Integrity\ Value\ (\%) = 100\ x\ \frac{(W_f)}{(W_i)}$$

Preparation of Cyclodextrin Complexes

The preparation of cyclodextrin complexes is described in the literature, and illustrative methods are incorporated herein for reference only.

A complex of γ-cyclodextrin and triclosan was formed by allowing a suspension of triclosan in an aqueous solution of γ-cyclodextrin (molar ratio 1.25 γ-cyclodextrin: 1.0 triclosan) to equilibrate for 24 hours at 25°C. with constant stirring. At equilibrium, a dense white precipitate corresponding to a 1:1 stoichiometric ratio of γ-cyclodextrin to triclosan was formed.

A complex of citral in β-cyclodextrin was prepared by mixing 200ml water and 62gm β-cyclodextrin at room temperature. Citral (7.6gm) was added dropwise to the suspension of β-cyclodextrin. After intensive stirring at room temperature for about 10 hours, the suspension was allowed to stand for a further 24 hours. The complex was filtered and vacuum dried at 40°C.

A complex of citral in γ-cyclodextrin was prepared by dissolving 70gm γ-cyclodextrin in 200ml water at 50°C, and adding citral (7.6gm) dropwise to the γ-cyclodextrin solution. After intensive stirring at 60°C for about 6 hours, the solution was allowed to stand for a further 24 hours. The complex was filtered and vacuum dried at 40°C. The concentration of citral in the γ-cyclodextrin complex was 11wt%.

A complex of evening primrose oil in γ-cyclodextrin at a level of 15wt% oil was prepared by dissolving 70gm γ-cyclodextrin in 200ml water at 45°C, and adding the evening primrose oil dropwise to the γ-cyclodextrin solution. Stirring was continued for 6 hours, and the complex was allowed to cool and stand for a further 24 hours, after which time it was filtered and dried in vacuo.

The invention will now be further described with reference to the following examples.

Examples 1 - 4

These examples illustrate a cyclodextrin containing hydrocolloid adhesive suitable for a WC flushable ostomy pouch. This flushable requirement means that the hydrocolloid should not be integrated, so that it will disintegrate satisfactorily in the sewage system. Example 1 is a hydrocolloid containing polyisobutylene, pectin, gelatine and sodium carboxymethyl cellulose, which was made as a control material. This hydrocolloid, described in US Patent 3,339,546, is an inelastic, non-integrated hydrocolloid adhesive which has been on the market as an ostomy barrier material and as a wound dressing material for many years, and is considered a standard product.

Each formulation was prepared in a 500gm batch in a 1l. Z-blade mixer. The pectin, the sodium carboxymethyl cellulose and the third powder was added to the mixer at 90°C and the powders blended together for 2 minutes. Then the Vistanex LMMH was added to the powders and the formulations were further mixed for 30 minutes at 90°C. The finished hydrocolloid adhesives were made into sheets of approximately 1mm thickness by pressing about 130gm of each formulation between two sheets of silicone release paper in a hydraulic press at 90°C.

| Wt% of Each Raw Material | Ex. 1 | Ex. 2 | Ex. 3 | Ex. 4 |
|---|---|---|---|---|
| Polyisobutylene, Vistanex LMMH | 40.6 | 40.6 | 40.6 | 50.0 |
| GenuPectin USP 100 | 19.8 | 19.8 | 19.8 | - |
| Sodium CMC, Blanose 7H4XF | 19.8 | 19.8 | 19.8 | - |
| Gelatine 100 mesh, 225 Bloom | 19.8 | - | - | - |
| β-Cyclodextrin (Cavitron 82000) | - | 19.8 | - | - |
| Hydroxypropyl β-Cyclodextrin (Cavitron 82005) | - | - | 19.8 | 50.0 |
| Totals, wt% | 100 | 100 | 100 | 100 |

| Physical Data | Ex. 1 | Ex. 2 | Ex. 3 | Ex. 4 |
|---|---|---|---|---|
| Reverse Tack, N/25mm | 17.5 | 21.5 | 20.3 | 18.7 |
| Peel Adhesion, 90°/ Stainless Steel, N/25mm | 8.8 | 7.9 | 12.0 | 15.0 |
| Shear, 0.5kg, minutes | 94 | 68 | 58 | 43 |
| Thickness,mm | 1.27 | 1.20 | 1.06 | 1.00 |
| Static Absorption, gm/sq.m./24hr | 7280 | 7242 | 7301 | 177 |
| Cold Flow, % increase/24hr/10kg | 7.2 | 18.7 | 11.7 | 18.6 |
| Integrity, %, 6hr | 65 | 82 | 58 | 97 |

Example 5

The hydrocolloid adhesive of Example 3 is laminated to a spun laced nonwoven fabric which had previously been waterproofed and coated with a medical grade acrylic pressure sensitive adhesive. The acrylic adhesive functions as a tie layer to bond the hydrocolloid adhesive to the polyester fabric. Such an acrylic adhesive coated nonwoven fabric is available commercially from Smith & Nephew plc as Lasso SA72.

The skin barrier so produced is made up into a drainable ostomy pouch having a stoma hole size of 32mm. The stoma pouch is used by a 22 year old ileostomy patient who is experiencing leakage problems with his commercially available pouch. The hydrocolloid adhesive of Example 3 has a high cold flow value, which means that the adhesive flows easily into the scarred depression on the patient's abdomen close to the stoma.

Although the hydrocolloid on the stoma erodes somewhat within 24 hours, the pouch adheres well, and the adhesive absorbs the odour caused by faecal contamination at the edge of the adhesive exposed close to the stoma opening. The patient is pleased with the performance of the pouch, and states that there is less odour noticeable with the pouch of example 5 compared to his current commercially available pouch.

Examples 6 - 8

Examples 6 - 8 show the effect of substituting cyclodextrins for one ingredient in a moderately integrated hydrocolloid formulation suitable for use as a hydrocolloid wound dressing. Aqualon A-500, which is a crystalline sodium carboxycellulose, was substituted with cyclodextrin material. 500gm batches of each formulation were prepared. The polyisobutylene (Vistanex LMMH), the Pectin, the Blanose sodium CMC and the third powder were added at 90°C to a 11. Z-blade mixer. After mixing for 15 minutes at 90°C, the temperature was raised to 100°C, and the other ingredient, the preformulated hot melt adhesive, was added and mixed for a further 30 minutes.

The Kraton KD-1161NS is a blend of linear styrene/isoprene/styrene triblock copolymer and linear styrene/isoprene diblock copolymer. Such a material is available from Shell Chemical Company and has a bound styrene content of about 15% and a diblock content of 17%. The mixture of tackifying resins used was a cyclopentadienyl resin, Escorez 2203LC, available from Exxon Chemical, and Adtac LV-E, a C5 synthetic hydrocarbon resin available from Hercules Chemical Company. The Irgafos 168 is an organophosphite stabiliser available from Ciba while the Irganox 565 is a hindered phenolic antioxidant also available from Ciba.

| Wt% of Each Raw Material | Ex 6 | Ex 7 | Ex 8 |
|---|---|---|---|
| Polyisobutylene, Vistanex LMMH | 28.0 | 28.0 | 28.0 |
| GenuPectin USP 100 | 14.0 | 14.0 | 14.0 |
| Sodium CMC, Blanose 7H4XF | 14.0 | 14.0 | 14.0 |
| Sodium CMC, Aqualon A-500 | 14.0 | - | - |
| β-Cyclodextrin (Cavitron 82000) | - | 14. 0 | - |
| Hydroxypropyl β-Cyclodextrin (Cavitron 82005) | - | - | 14.0 |
| Kraton D-1161NS | 11.3 | 11.3 | 11.3 |
| Adtac LV-E | 6.0 | 6.0 | 6.0 |
| Escorez 2203 LC | 12.5 | 12.5 | 12.5 |
| Irgafos 168 | 0.14 | 0.14 | 0.14 |
| Irganox 565 | 0.07 | 0.07 | 0.07 |
| Total | 100 | 100 | 100 |

| Physical Data | Ex 6 | Ex 7 | Ex 8 |
|---|---|---|---|
| Reverse Tack, N/25mm | 27.3 | 34. 6 | 23.7 |
| Peel Adhesion, 90°/SS, N/25mm | 12.7 | 16. 0 | 16.6 |
| Shear, 0.5kg, minutes | 129 | 190 | 237 |
| Thickness, mm | 0.67 | 0.7 5 | 0.70 |
| Static Absorption, gm/sq.m./24hr | 6265 | 378 9 | 4648 |
| Cold Flow, % increase/24hr/10kg | 0.8 | 4.7 | 3.5 |
| Integrity, %, 24hr. | 50 | 78 | 74 |

Examples 9 - 11

These examples demonstrate the odour absorbing properties of adhesives made according to the teachings herein. An adhesive, Example 9 to be used as a control in the following experiments and containing no cyclodextrins, was made according to our copending Application number PCT No: GB98/02809 and Examples 10 and 11 were made according to the teachings herein. The compositions of the three adhesives are given in the Table below:

| Amount in Mix, wt% | Example 9 | Example 10 | Example 11 |
|---|---|---|---|
| Kraton D-1161NS | 11.3 | 11.3 | 5.9 |
| Adtac LV-E | 6.0 | 6.0 | - |
| Escorez 2203 LC | 12.5 | 12.5 | - |
| Irgafos 168 | 0.14 | 0.14 | - |
| Irganox 565 | 0.07 | 0.07 | - |
| Vistanex LMMH | 28 | 28 | 28 |
| LVSI 101 | - | - | 23.7 |
| Irganox 1010 | - | - | 0.4 |
| Cavitron 82000 | - | 14 | - |
| β-Cyclodextrin W7 | - | - | 14 |
| Methyl substituted β-Cyclodextrin W7M1.8 | - | - | 14 |
| Sodium CMC | 14 | 14 | - |
| Pectin USP100 | 14 | 14 | - |
| Aquasorb A-500 | 14 | - | 14 |

The three adhesives were pressed to a thickness of about 1mm between two pieces of release paper using a hydraulic press held at 90°C. The sheets were then laminated to 25μ polyurethane film, previously coated with a medical grade acrylic adhesive to act as a tie coat between the film and the hydrocolloid adhesive.

Using the MVT cups described above under the Section "Test methods - Static absorption of hydrocolloids", the pressed and laminated samples from Examples 9 - 11 were adhered to the flanges of cups that contained 30 ml aqueous NaCl solution (0.9%wt). Exactly as described in the Test method, the cups were turned upside down, put in the oven at 37°C. for 24 hours and then cooled down. The hydrocolloids were removed from the cups, the circular saline-saturated portions were cut out from the remainder of the hydrocolloid pad, and each circle was placed in the bottom of a wide-mouthed screw-capped 1l. jar, hydrocolloid side up.

Each of the saline soaked pads was inoculated with 5μl of n-butyric acid using a microsyringe, and the caps screwed tightly on the jars. The n-butyric acid is a strong smelling compound found, together with other fatty acids, in odiferous wounds. After 24 hours, the three samples were smelled in turn by four panelists, who rated each of the three samples according to intensity of odour.

First, the panelists smelled each jar and ranked them according to strength of odour, from strongest odour to weakest. The panelists then smelled each jar a second time, and scored each on a scale of 0-5, 0 being no smell and 5 being the strongest.

These ratings of 0-5 for each adhesive were then rank ordered among the adhesives for each panelist, and the rankings for each adhesive were summed among the panelists. The following data were obtained, where 12 is the maximum possible score (highest odour), and 4 is the lowest possible score (lowest odour).

| Sample | Odour Level, Sum of Rankings, Σ |
|---|---|
| Example 9 | 12 |
| Example 10 | 7 |
| Example 11 | 5 |

The data showed that little or no odour of n-butyric acid was detected with the adhesives of Examples 10 and 11, while the adhesive from Example 9, which contains no cyclodextrin material, retained the strong, unpleasant odour of n-butyric acid after 24 hours.

Example 12

This example shows the incorporation of cyclodextrins into a pressure sensitive adhesive formed from a hydrogel.

A glass vessel of 250ml capacity is tared on an electronic balance and polyvinyl alcohol (0.1gm) is added, followed by 5ml of deionised water. γ-cyclodextrin (0.5gm) and α-cyclodextrin (0.5gm) are then added and the mixture is stirred vigorously until all the solid materials are dissolved. The following monomers are then introduced into the glass vessel: N,Ndimethyl acrylamide (10gm), methoxypolyethylene glycol methacrylate (MW400, 1gm), polyethylene glycol dimethacrylate (0.10gm), Daracure 1173 (CIBA, 0.4gm) and 1:2 propylene glycol (1gm).

The ingredients are mixed thoroughly and coated in a thin layer on to a piece of silicone release paper. The coated paper is passed 12 times beneath a FUSION F300S UV curing apparatus on a conveyor belt running at a speed of about 4m/min. The bulb used is 15cm long and emits light of wavelength λ in the range 200 - 400nm.

A self-supporting sheet of aqueous tacky gel is produced, which is flexible enough to be removed from the release paper, and which shows good adhesion to dry skin.

## Claims

1. A medical or surgical appliance in the form of a wound dressing, an ostomy appliance or a skin barrier, comprising a substrate on which is formed a layer of pressure sensitive adhesive composition, the pressure sensitive adhesive composition comprising a rubbery continuous phase with a discontinuous phase distributed therein, **characterised in that** the discontinuous phase comprises 0.1 to 65 wt %, based on the total composition, of a hydrocolloid composition comprising an uncomplexed cyclodextrin, and a hydrocolloid other than cyclodextrin.

2. An appliance according to claim 1 wherein the cyclodextrin is present in an amount of at least 5 wt % based on the total composition.

3. An appliance according to claim 2 wherein the cycoldextrin is present in an amount of at least 10 wt % based on the total composition.

4. An appliance according to any preceding claim wherein the continuous phase comprises up to 50 wt % of polyisobutylene as a major component.

5. An appliance according to any preceding claim wherein the continuous phase contains up to 15 wt % of a rubbery copolymer of styrene or a substituted styrene as a major component.

6. An appliance according to any preceding claim wherein said hydrocolloid combined with the cyclodextrin is selected from pectins, guar gum, locust bean gum, karaya gum, carageenan, tragacanth gum, alginates, carboxymethyl cellulose, polyvinylalcohol, polyoxyalkylene polyols, polyvinyl pyrrolidone gelatine, hyaluronic acid, chitosan salts and DEAE dextran.

7. An appliance according to any preceding claim wherein the adhesive composition contains an active ingredient.

8. An appliance according to claim 7 wherein the active ingredient is selected from hydroquinone, menthol, salicylic acid, antibacterial agents, antifungal agents, essential oils and fragrances.

9. An appliance according to any preceding claim wherein the substrate includes a backing comprising non-adhesive waterproof film.

## Patentansprüche

1. Eine medizinische oder chirurgische Vorrichtung in Form eines Wundverbandes, einer Stomavorrichtung oder einer Hautbarriere, die ein Substrat aufweist, auf dem eine Schicht aus einer Kontaktklebstoffzusammensetzung gebildet ist, wobei die Kontaktklebstoffzusammensetzung eine gummiartige kontinuierliche Phase mit einer darin verteilten diskontinuierlichen Phase aufweist, **dadurch gekennzeichnet, dass** die diskontinuierliche Phase bezogen auf die gesamte Zusammensetzung 0,1 bis 65 Gew.-% einer Hydrokolloidzusammensetzung aufweist, die ein nicht komplexiertes Cyclodextrin und ein Hydrokolloid aufweist, das kein Cyclodextrin ist.

2. Vorrichtung nach Anspruch 1, worin das Cyclodextrin in einer Menge von zumindest 5 Gew.-% bezogen auf die gesamte Zusammensetzung vorliegt.

3. Vorrichtung nach Anspruch 2, worin das Cyclodextrin in einer Menge von zumindest 10 Gew.-% bezogen auf die gesamte Zusammensetzung vorliegt.

4. Vorrichtung nach einem der vorhergehenden Ansprüche, worin die kontinuierliche Phase bis zu 50 Gew.-% Polyisobutylen als einen Hauptbestandteil aufweist.

5. Vorrichtung nach einem der vorhergehenden Ansprüche, worin die kontinuierliche Phase bis zu 15 Gew.-% eines gummiartigen Copolymers von Styrol oder einem substituierten Styrol als einen Hauptbestandteil enthält.

6. Vorrichtung nach einem der vorhergehenden Ansprüche, worin das mit dem Cyclodextrin kombinierte Hydrocolloid ausgewählt ist aus den Pectinen, Guarmehl, Johannisbrotkernmehl, Karaya-Gummi, Carageenan, Tragantgummi, den Alginaten, Carboxymethylcellulose, Polyvinylalkohol, den Polyoxyalkylenpolyolen, Polyvinylpyrrolidon, Gelatine, Hyaluronsäure, den Chitosansalzen und DEAE-Dextran.

7. Vorrichtung nach einem der vorhergehenden Ansprüche, worin die Klebstoffzusammensetzung einen Wirkstoff enthält.

8. Vorrichtung nach Anspruch 7, worin der Wirkstoff ausgewählt ist aus Hydroquinon, Menthol, Salicylsäure, den antibakteriellen Mitteln, den antimykotischen Mitteln, den ätherischen Ölen und den Aromen.

9. Vorrichtung nach einem der vorhergehenden Ansprüche, worin das Substrat einen Träger einschließt, der eine nicht klebende wasserdichte Folie aufweist.

**Revendications**

1. Dispositif médical ou chirurgical sous la forme d'un pansement pour blessure, d'un appareillage stomique ou d'une barrière de protection pour la peau, comprenant un substrat sur lequel est formée une couche de composition adhésive sensible à la pression, la composition adhésive sensible à la pression comprenant une phase continue caoutchouteuse ayant une phase discontinue dispersée dans celle-ci, **caractérisé en ce que** la phase discontinue comprend 0,1 à 65 % en poids, sur la base de la composition totale, d'une composition à base d'hydrocolloïde comprenant une cyclodextrine non complexée, et un hydrocolloïde autre que la cyclodextrine.

2. Dispositif selon la revendication 1, dans lequel la cyclodextrine est présente en quantité égale à au moins 5 % en poids sur la base de la composition totale.

3. Dispositif selon la revendication 2, dans lequel la cyclodextrine est présente en quantité égale à au moins 10 % en poids sur la base de la composition totale.

4. Dispositif selon l'une quelconque des revendications précédentes, dans lequel la phase continue comprend jusqu'à 50 % en poids de polyisobutylène comme composant principal.

5. Dispositif selon l'une quelconque des revendications précédentes, dans lequel la phase continue comprend jusqu'à 15 % en poids d'un copolymère caoutchouteux de styrène ou d'un styrène substitué comme composant principal.

6. Dispositif selon l'une quelconque des revendications précédentes, dans lequel ledit hydrocolloïde combiné avec la cyclodextrine est choisi parmi les pectines, la gomme de guar, la gomme de caroube, la gomme de karaya, le carraghénane, la gomme adragante, des alginates, la carboxyméthylcellulose, l'alcool polyvinylique, les polyols de polyoxyalkylène, la gélatine, la polyvinylpyrrolidone, l'acide hyaluronique, les sels de chitosane et le dextrane DEAE.

7. Dispositif selon l'une quelconque des revendications précédentes, dans lequel la composition adhésive contient un ingrédient actif.

8. Dispositif selon la revendication 7, dans lequel l'ingrédient actif est choisi parmi l'hydroquinone, le menthol, l'acide salicylique, des agents antibactériens, des agents antifongiques, des huiles essentielles et des parfums.

**9.** Dispositif selon l'une quelconque des revendications précédentes, dans lequel le substrat comprend un revêtement comprenant un film imperméable non adhésif.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9801167 A
- EP 0023395 B1
- US 3339546 A
- US 4231369 A
- US 4477325 A
- US 4738257 A
- US 4551490 A
- US 4192785 A
- US 4952618 A
- JP 6158002 A
- US 5352717 A
- US 4978532 A
- US 5562917 A
- WO 9856343 A
- WO 9856344 A
- WO 9856345 A
- WO 9906078 A
- WO 9856890 A
- WO 9817239 A
- WO 9817240 A
- WO 9856341 A
- WO 9856342 A
- WO 9818439 A
- WO 985634 A
- WO 9856339 A
- US 5429628 A
- WO 9422501 A
- EP 0392608 B1
- WO 9418973 A
- US 5246611 A
- WO 9305136 A
- WO 9117300 A
- JP 10168348 A
- US 93168550 A
- DE 19724871 A1
- US 5399404 A
- WO 9423766 A1
- CA 2041278 A
- GB 9802809 A